# EUROPEAN PATENT SPECIFICATION

(11) **EP 1 264 184 B1**
(45) Date of publication and mention of the grant of the patent: **04.01.2006**
(21) Application number: 01925453.1
(22) Date of filing: 15.03.2001
(51) Int. Cl.: G01N 33/68, G01N 33/58, C12Q 1/28

(54) **MOLECULAR WEIGHT MARKERS FOR WESTERN BLOT**
MOLEKULARGEWICHTSMARKER FÜR WESTERN BLOT
ETALONS DE MASSE MOLECULAIRE POUR TRANSFERT DE TYPE WESTERN

(30) Priority: 17.03.2000 IT MI2000 552
(43) Date of publication of application: 11.12.2002
(73) Proprietor: Dompe' S.P.A., 67100 L'Aquila (IT)
(72) Inventor: RUGGIERO, Paolo, I-67100 L'Aquila (IT); MAURIZI, Giovanni, I-67100 L'Aquila (IT); CIABINI, Annibale, I-67100 L'Aquila (IT); DI CIOCCIO, Vito, I-67100 L'Aquila (IT)
(74) Representative: Minoja, Fabrizio
(86) International application number: PCT/EP2001/002890
(87) International publication number: WO 2001/068899

(56) References cited:
- DE-C- 19 729 248
- US-A- 4 302 534
- MAURIZI GIOVANNI ET AL: "Peroxidase-active molecular weight markers for direct detection in Western blot." ANALYTICAL BIOCHEMISTRY, vol. 286, no. 2, 15 November 2000 (2000-11-15), pages 295-296, XP002175929 ISSN: 0003-2697
- NEUMAIER M ET AL: "TRANSBLOT STUDIES WITH BIOTIN-LABELED PROTEINS ELECTROPHORETIC MOBILITIES AND DETECTION LIMITS" ANALYTICAL BIOCHEMISTRY, vol. 156, no. 1, 1986, pages 76-80, XP001016253 ISSN: 0003-2697
- DELLA-PENNA D ET AL: "BIOTINYLATED PROTEINS AS MOLECULAR WEIGHT STANDARDS ON WESTERN BLOTS" ANALYTICAL BIOCHEMISTRY, vol. 152, no. 2, 1986, pages 329-332, XP001016252 ISSN: 0003-2697
- VARGAS C. ET AL.: "Detection of C-type cytochromes using enhanced chemiluminescence" ANALYTICAL BIOCHEMISTRY, vol. 209, 1993, pages 323-326, XP002175930 cited in the application

## Description

The present invention generally relates to the techniques for the separation and detection of proteins by electrophoresis and western blot. More particularly, the invention relates to a method for the preparation of molecular weight markers (standards) for use in western blot.

### TECHNOLOGICAL BACKGROUND

Since the introduction of western blot technique, radioisotope-labelled antibodies have been progressively replaced by the enzyme-labelled antibodies, mainly for their easier handling and shorter detection time. At present, the most popular methods for western blot detection of proteins are based on peroxidase conjugates (antibodies, protein A, protein G, avidin, streptavidin, and the like) whose enzymatic activity is revealed by an appropriate substrate, either chromogenic or chemiluminescent.

In common laboratory procedure, the molecular weight standards used in western blot, after transfer on membrane and staining, are marked by pencil to exactly attribute the corresponding bands. This procedure is also followed when using pre-stained standards, as the colors are generally altered or attenuated at the end of the western blot procedure, and therefore some bands disappear or are hardly detectable on the membrane. Moreover, when using chemiluminescent methods, all these standards have to be marked again on the autoradiography film, to avoid uncertain determination of the detected band.

Molecular weight standards directly detectable on the membrane exist, but they require specific conditions or treatments. For example, biotin-conjugated standards are only detectable when biotin-(strept)avidin systems are used. A set of standards sharing a common epitope, detectable by a specific antibody included within the secondary antibody solution, is commercially available (Santa Cruz Biotechnology, Santa Cruz, CA, U.S.A.). However, this method implies purchasing both markers and secondary antibodies from a unique manufacturer. No standards detectable by the peroxidase enzymatic activity directly in the membrane used for western blot are currently available.

Methods for the detection of heme-proteins, after SDS-PAGE and transfer onto a nitrocellulose filter, based on the use of peroxidase substrates, both chromogenic and chemiluminescent, have been reported (Dorward D.W., 1993, Anal. Biochem. 209:219; Vargas C. et al., 1993, Anal. Biochem. 209:323). Furthermore, electrophoresis standards obtained by polymerization of a protein that gives raise to a discrete series of homopolymers are commercially available (Sigma, U.S.A., catalogue A9392, H2757, H2507, P8906).

### DISCLOSURE OF THE INVENTION

It has now been found that the peroxidase activity can be retained and, at least partially, stabilized against denaturation by conjugation of two or more proteins having peroxidase activity or of proteins having peroxidase activity with other proteins without peroxidase activity. This allowed to prepare molecular weight standards which can directly be detected on the membrane used for western blot by reaction with a suitable peroxidase substrate.

Therefore, the invention relates to a method for the preparation of molecular weight standards for use in western blot, comprising the conjugation of two or more (poly)peptides, that can be the same or different, at least one of which has peroxidase activity, by a cross-linker, and, optionally, the subsequent separation of the conjugation products so as to cover the desired molecular weight range.

"Conjugation" herein means the link of two or more (poly)peptides by means of a suitable compound (cross-linker) capable of covalently binding the two (poly)peptides. Said cross-linkers can be selected from the group consisting of all the irreversible bifunctional cross-linkers. Disuccinimidyl suberate (DSS) or its water-soluble analog disuccinimidyl glutarate (DSG) are preferably used.

"Proteins having peroxidase activity" herein preferably means the proteins or (poly)peptides having an heme group. Cytochrome C, microperoxidase, lactoperoxidase and horseradish peroxidase are non-limitative examples of proteins having peroxidase activity which can be used according to the invention.

On the other hand, "protein without peroxidase activity" can be any non-heme protein with known molecular weight having constant, reproducible migration under the different conditions used for the electrophoresis, and capable of being easily and stably conjugated with the above mentioned cross-linker. The non-heme protein is preferably selected from the group consisting of lysozyme, carbonic anhydrase, ovalbumin and serum albumin.

Conjugation can be either single, i.e. two equal or different proteins are joined, or multiple, i.e. more than two proteins may be variously joined by means of the cross-linker. Conjugation multiplicity can be adjusted by varying the conditions of the reaction between the cross-linker and the protein or mixture of different proteins, particularly the reaction time or the reagent concentration. After completion of the reaction, a mixture of products with different conjugation multiplicities will be obtained. The conjugation provides different products, the amount of each being inversely related to its molecular weight. The product mixture can be used either as such or separated into its different components. In this way, standards covering various molecular weight ranges can be prepared. In order to define more accurately the molecular weight, for each preparation a calibration may be performed by SDS-PAGE and/or western blot or with a commercial or laboratory standard consisting of proteins with known molecular weight. It should however be considered that western blot is not an analytical technique for the determination of molecular weight. Therefore, standards whose molecular weight is not precisely determined can be used, as it is commonly the case with commercial pre-stained standards, where conjugation with the chromophore molecule induces an alteration in the electrophoretic migration.

The standards, after electrophoretic separation and transfer on the western blot membrane, can be directly detected on the membrane by reaction with a chromogenic substrate or on the autoradiography film, when using chemiluminescent substrates. Examples of conventional chromogenic and chemiluminescent substrates are: 4-chloro-1-naphthol and diaminobenzidine (chromogenic); ECL (Amersham), Supersignal (Pierce) and DuoLux (Vector) (chemiluminescent). Since cross-linking can prevent the complete protein denaturation under SDS-PAGE conditions, resulting in unpredictable migration, the apparent M.W. of the labeled proteins should preferably be estimated by calibration with commercial markers.

Furthermore, in the case of conjugation products between different proteins, the protein having peroxidase activity should preferably have low molecular weight, to increase the conjugation (peroxidase/protein) ratio thus providing better detection of the standards. Microperoxidases are preferred, such as microperoxidase MP-11, a cytochrome C peptide having 11 amino acids, or microperoxidases MP-9 and MP-8, respectively nona- or octapeptide, described by Plattner et al., 1977, *Histochemistry* 53:223-242, or by Harbury et al., 1960. *J*. *Biol. Chem*., 235:3649-3655, or by Kraehenbuhl et al., 1974, *J. Exp*. *Med*., 139:208-223; furthermore, MP-6 and MP-17, having 6 and 17 amino acids respectively, (Spee et al, 1996, Eur. J. Biochem. 241:215-220), can be used. As a rule, any peptide derived from peroxidase proteins by proteolysis or synthesis can be used, as far as it retains its enzymatic activity.

In principle, the molecular weight standards of the invention proved to be stable after treatment in SDS-PAGE and western blot, although the peroxidase activity is better preserved under mild denaturing conditions, avoiding high-temperature.

A further aspect of the invention relates to a kit for the preparation of molecular weight standards including, in separate containers, the protein/peptide having peroxidase activity, the cross-linker and optionally the protein having no peroxidase activity, the incubation buffer and the buffer to stop the reaction. The buffers can be Tris or lysine buffer, lysine buffer being preferred in that it provides a better protein-membrane interaction.

The effectiveness of small (poly)peptides having peroxidase activity as protein markers allows their use in place of more hindering peroxidase proteins. Secondary antibodies, avidin or streptavidin usually employed in western blot, E.L.I.S.A, immunocytochemistry and immunohistochemistry, are examples of molecules that can preferably be conjugated with the microperoxidase. The resulting conjugate will have a remarkably reduced size and a higher per-molecule number of markers, thus increasing its efficiency.

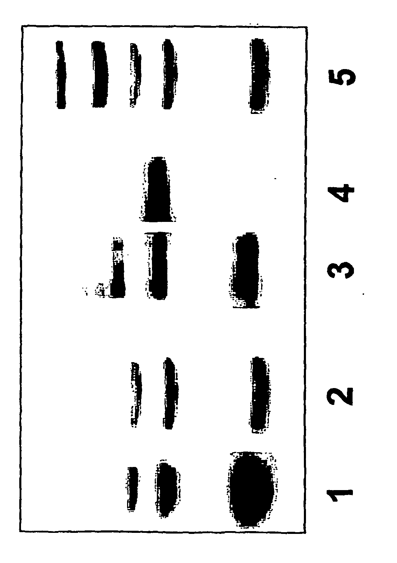

### DESCRIPTION OF THE FIGURE

- lane 1: (from bottom to top) cytochrome C monomer, dimer and trimer;
- lane 2: as in lane 1, after separating and mixing the oligomers to give bands of comparable intensity;
- lane 3: lysozyme conjugated with microperoxidase (from bottom to top) monomer, dimer, trimer;
- lane 4: carbonic anhydrase conjugated with microperoxidase;
- lane 5: (from bottom to top) cytochrome C monomer, dimer and trimer as in lane 2; ovalbumin conjugated with cytochrome C; bovine serum albumin conjugated with cytochrome C.

The following examples illustrate the invention in greater detail.

### EXAMPLES

### EXAMPLE 1: ~ 13 to 40 kDa range (cytochrome C oligomers)

Material:
20 mg cytochrome C
buffer A: 10 mM NaPᵢ pH 7.4, 150 mM NaCl
buffer B: 1M DL-lysine in buffer A
buffer C: 40 mM Tris-Cl pH 7.4, 300 mM NaCl
solution D: 20 mM DSS in dimethyl sulfoxide (extemporary preparation).
   1. Dissolve cytochrome C in 0.18 ml of buffer A.
   2. Add 0.02 ml of solution D.
   3. Stir 1h at room temperature.
   4. Add 0.02 ml of buffer B.
      If a set with bands having the same intensity is desired, continue with the subsequent steps, otherwise directly skip to step 7.
   5. Load on a Superdex 75 column (10 x 300 mm) or on a column with similar characteristics, equilibrated and eluted in buffer C at a 0.5 ml/min flow rate, separately recovering the eluted peaks.
   6. Mix each peak in amounts inversely proportional to the chromatographic peak area.
   7. Aliquot and freeze at a temperature below-20°C.

### EXAMPLE 2: ~ 60 to 130 kDa range (cytochrome C conjugates)

Material:
1 mg cytochrome C
3.3 mg ovalbumin
10 mg bovine serum albumin
buffer A: 10 mM NaPᵢ pH 7.4, 150 mM NaCl
buffer B: 1M DL-lysine in buffer A
solution D: 20 mM DSS in dimethyl sulfoxide (extemporary preparation).
   1) Dissolve cytochrome C in 1 ml of buffer A.
   2) Dissolve ovalbumin in 0.8 ml of buffer A and add 0.1 ml of solution from step 1.
   3) Dissolve bovine serum albumin in 0.8 ml of buffer A and add 0.1 ml of solution from step 1.
   4) Add 0.1 ml of solution D to the solutions prepared at steps 2 and 3.
   5) Stir the solutions from step 4 for 1 h at room temperature.
   6) Add 0.1 ml of buffer B.
   7) Aliquot and freeze at temperature below -20° C.

### EXAMPLE 3: ~ 15 to 45 kDa range (microperoxidase MP-11 conjugates)

Material:
1 mg microperoxidase MP-11
10 mg lysozyme
5 mg carbonic anhydrase
buffer A: 10 mM NaPᵢ pH 7.4, 150 mM NaCl
buffer B: 1M DL-lysine in buffer A
solution D: 20 mM DSS in dimethyl sulfoxide (extemporary preparation).
   1. Dissolve microperoxidase in 1 ml of buffer A.
   2. Dissolve lysozyme in 0.8 ml of buffer A and add 0.1 ml of solution from step 1.
   3. Dissolve carbonic anhydrase in 0.8 ml of buffer A and add 0.1 ml of solution of step 1.
   4. Add 0.1 ml of solution D to the solutions prepared at steps 2 and 3.
   5. Stir the solutions from step 4 for 1 h at room temperature.
   6. Add 0.1 ml of buffer B.
   7. Aliquot and freeze at temperature below - 20°C.

### EXAMPLE 4 - Western Blot

Duplicated samples of unconjugated cytochrome C, horseradish peroxidase, lactoperoxidase, and of conjugated proteins (with both cytochrome C and MP-11) were mixed with reducing Laemmli sample buffer: the first duplicate was heated for 5 min at 95°C, the second at 40°C, then the samples were run in parallel with Broad Molecular Weight Markers (Bio-Rad, Hercules, CA, U.S.A.) on 15% mini-SDS-PAGE following standard procedure. After run, the gel was blotted onto 0.45 µm nitrocellulose sheets by a semi-dry cell. Membranes were washed with 20 mM TrisCl pH 7.4, 150 mM NaCl (TBS), blocked 1 h in 3% (w/v) bovine serum albumin in TBS (BT), then incubated in BT containing 0.05% (v/v) Tween-20 either 2 h or overnight to simulate different western blot procedures. After washing in TBS containing 0.25% Tween-20 and further washing in TBS, the peroxidase activity was revealed.

Cytochrome C was also treated with DSS without adding any other protein, and subjected to gel-filtration on a Superdex 75 (1 x 30 cm) column (Amersham Pharmacia Biotech, Uppsala, Sweden) equilibrated and eluted in 40 mM Tris-Cl pH 7.4, 300 mM NaCl, at 0.5 ml/min flow: each eluted peak was recovered separately.

Cytochrome C and MP-11 conjugates were found to retain peroxidase activity at the end of the western blot procedure, as summarized in Table 1A-B. Unconjugated peroxidase proteins were found to be more sensitive to high temperature treatment, which drastically decreased their enzymatic activity: cross-linker treatment is in fact likely to stabilize the protein structure against denaturation.

### TABLE 1

**A. Cytochrome C conjugates**

| | | | Cytochrome C | | | |
|---|---|---|---|---|---|---|
| | | | mg/ml | | | |
| | | | 100 | 10 | 0.1 | |
| Protein and source | KDa | Mg/ml | *(a)* | | | Approximate protein/cytochrome C molar ratio |
| Cytochrome C (Sigma C.2506 | 12.04 | 100 | + | | | |
| | | 10 | | + | | |
| Ribonuclease I A (Pharmacia 27-0323-01) | 13.7 | 1 | | | - | 10 |
| Ovalbium (Sigma A-7642) | 45. | 3.3 | | | + | 10 |
| Bovine serum albumin (Bio-Rad 500-0007) | 67. | 10 | | | + | 20 |

**B. MP11 microperoxide conjugates**

| | | | MP-11 (Sigma M-6756 1.8 kDa 0.1mg/ml) | |
|---|---|---|---|---|
| Protein and source | KDa | Mg/ml | *(a)* | Approximate Protein/MP-11 Molar Ratio |
| Lysozyme (Sigma L-6876) | 14.3 | 10 | + | 12 |
| Carbonic anhydrase (Sigma C-2273) | 29 | 5 | + | 3 |

| | | | | |
|---|---|---|---|---|
| *(a)* the sign + o - indicates whether after western blot procedure the peroxidase activity of the conjugate was detectable or not, respectively | | | | |

When cytochrome C was treated with the cross-linker, three oligomers were generated (Figure, lanes 1 and 2) which were easily separated by gel filtration. The electrophoretic mobility suggests their identification as monomers, dimers and trimers (Table 2).

The amount of each conjugate and of the cytochrome C oligomers was adjusted so as to make the signal intensity of each band as homogeneous as possible. Mixing the three species cytochrome C, ovalbumin- and serum albumin-cytochrome C, provided a valuable series of molecular weight standards (Figure, lane 5). The conjugation of MP-11 with lysozyme produced three species of lysozyme oligomers, all of them MP-11 conjugates (Table 1B and Figure, lane 3), monomers, dimers and trimers (Table 2). Conjugation of MP-11 with carbonic anhydrase produced a single band (lane 4).

**TABLE 2 Calibrated molecular weights of the conjugates**

| | KDa |
|---|---|
| Cytochrome C, monomer | 12.9 |
| Cytochrome C, dimer | 28.4 |
| Cytochrome C, trimer | 38.6 |
| Lysozyme-MP-11, monomer | 14.8 |
| Lysozyme-MP-11, dimer | 30.7 |
| Lysozyme-MP-11, trimer | 45.5 |
| Carbonic anhydrase -MP11 | 31.5 |
| Ovalbumin-cytochrome C | 58.9 |
| Bovine serum albumin-cytochrome C | 131 |

## Claims

1. A method for the preparation of molecular weight standards for western blot, which comprises conjugating two or more peptides or polypeptides, which can be the same or different, at least one of them having peroxidase activity and being selected from cytochrome C and microperoxidase, by means of a cross-linker capable of covalently binding said (poly)peptides.

2. The method as claimed in claim 1, further comprising the separation of the conjugation products according to their molecular weights.

3. A method as claimed in claims 1-2, wherein the protein without peroxidase activity is selected from the group comprising lysozyme, carbonic anhydrase, ovalbumin and serum albumin.

4. A method as claimed in any one of the preceding claims, wherein the cross linker is selected from irreversible bifunctional cross-linkers.

5. Molecular weight standards for western blot containing a conjugation product of two or more peptides or polypeptides, which can be the same or different, at least one of them having peroxidase activity and being selected from cytochrome C and microperoxidase, by means of a cross-linker capable of covalently binding said peptides or polypeptides.

6. Molecular weight standards as claimed in claim 5, selected from the group consisting of cytochrome C conjugate, microperoxidase and lysozyme conjugate, carbonic anhydrase and microperoxidase conjugate, cytochrome C and ovalbumin conjugate, cytochrome C and serum albumin conjugate.

7. A kit for the preparation of the molecular weight standards as claimed in claims 5 and 6, comprising, in separate containers, the peptide or polypeptide having peroxidase activity and the cross-linker, and optionally the protein without peroxidase activity and the incubation buffer.

8. The use of cytochrome C or microperoxidase for the preparation of a molecular weight standard for western blot.

## Patentansprüche

1. Verfahren zur Herstellung von Molekulargewichtsstandards für Western Blot, das ein Konjugieren von zwei oder mehreren Peptiden oder Polypeptiden, die gleich oder verschieden sein können, wobei mindestens eines von diesen Peroxidase-Aktivität aufweist und aus Cytochrom C und Mikroperoxidase ausgewählt ist, mittels eines Vernetzungsmittels, das zum kovalenten Binden der (Poly)Peptide fähig ist, umfasst.

2. Verfahren wie in Anspruch 1 beansprucht, das ferner die Auftrennung der Konjugationsprodukte gemäß ihren Molekulargewichten umfasst.

3. Verfahren wie in den Ansprüchen 1 bis 2 beansprucht, wobei das Protein ohne Peroxidase-Aktivität aus der Gruppe ausgewählt ist, die Lysozym, Carboanhydrase, Ovalbumin und Serumalbumin umfasst.

4. Verfahren wie in einem der vorstehenden Ansprüche beansprucht, wobei das Vernetzungsmittel aus irreversiblen bifunktionellen Vernetzungsmitteln ausgewählt ist.

5. Molekulargewichtsstandards für Western Blot, die ein Konjugationsprodukt von zwei oder mehreren Peptiden oder Polypeptiden, die gleich oder verschieden sein können, wobei mindestens eines von diesen Peroxidase-Aktivität aufweist und aus Cytochrom C und Mikroperoxidase ausgewählt ist, mittels eines Vernetzungsmittels, das zum kovalenten Binden der Peptide oder Polypeptide fähig ist, enthalten.

6. Molekulargewichtsstandards wie in Anspruch 5 beansprucht, die aus der Gruppe ausgewählt sind, bestehend aus Cytochrom C-Konjugat, Konjugat aus Mikroperoxidase und Lysozym, Konjugat aus Carboanhydrase und Mikroperoxidase, Konjugat aus Cytochrom C und Ovalbumin, Konjugat aus Cytochrom C und Serumalbumin.

7. Kit zur Herstellung der Molekulargewichtsstandards wie in den Ansprüchen 5 und 6 beansprucht, umfassend in getrennten Behältern das Peptid oder Polypeptid mit Peroxidase-Aktivität und das Vernetzungsmittel und gegebenenfalls das Protein ohne Peroxidase-Aktivität und den Inkubationspuffer.

8. Verwendung von Cytochrom C oder Mikroperoxidase zur Herstellung eines Molekulargewichtsstandards für Western Blot.

## Revendications

1. Procédé de préparation de standards de poids moléculaire pour analyse western blot, qui comprend l'opération consistant à conjuguer au moins deux peptides ou polypeptides, qui peuvent être identiques ou différents, l'un au moins d'entre eux ayant une activité peroxydase et étant choisi entre le cytochrome C et une microperoxydase, au moyen d'un agent de réticulation capable de lier de manière covalente lesdits (poly)peptides.

2. Procédé tel que revendiqué dans la revendication 1, comprenant en outre la séparation des produits de conjugaison selon leurs poids moléculaires.

3. Procédé tel que revendiqué dans les revendications 1-2, dans lequel la protéine sans activité peroxydase est choisie dans le groupe comprenant un lysozyme, une anhydrase carbonique, une ovalbumine et une albumine de sérum.

4. Procédé tel que revendiqué dans l'une quelconque des revendications précédentes, dans lequel l'agent de réticulation est choisi parmi des agents de réticulation bifonctionnels irréversibles.

5. Standards de poids moléculaire pour analyse western blot, contenant un produit de conjugaison d'au moins deux peptides ou polypeptides, qui peuvent être identiques ou différents, l'un au moins d'entre eux ayant une activité peroxydase et étant choisi entre le cytochrome C et une microperoxydase, au moyen d'un agent de réticulation capable de lier de manière covalente lesdits peptides ou polypeptides.

6. Standards de poids moléculaire tels que revendiqués dans la revendication 5, choisis dans le groupe constitué par un conjugué de cytochrome C, un conjugué de microperoxydase et de lysozyme, un conjugué d'anhydrase carbonique et de microperoxydase, un conjugué de cytochrome C et d'ovalbumine, un conjugué de cytochrome C et d'albumine de sérum.

7. Kit pour la préparation de standards de poids moléculaire tels que revendiqués dans les revendications 5 et 6, comprenant, dans des conteneurs séparés, le peptide ou polypeptide ayant une activité peroxydase et l'agent de réticulation, et éventuellement la protéine sans activité peroxydase et le tampon d'incubation.

8. Utilisation du cytochrome C ou de la microperoxydase pour la préparation d'un standard de poids moléculaire pour analyse western blot.
